**Europäisches Patentamt**

**European Patent Office**

**Office européen des brevets**

(11) Veröffentlichungsnummer: **0 008 633**
**B1**

(12) **EUROPÄISCHE PATENTSCHRIFT**

(45) Veröffentlichungstag der Patentschrift:
**09.12.81**

(21) Anmeldenummer: **79102346.8**

(22) Anmeldetag: **09.07.79**

(51) Int. Cl.³: **F 28 D 7/02,** B 01 J 8/02,
B 21 D 53/02, C 07 C 9/04

(54) Wärmetauscher für Hochdruck- und Hochtemperatureinsatz und Verfahren zu seiner Herstellung sowie Verwendung als Reaktor.

(30) Priorität: **10.07.78 DE 2830225**
**30.01.79 DE 2903466**

(43) Veröffentlichungstag der Anmeldung:
**19.03.80 Patentblatt 80/6**

(45) Bekanntmachung des Hinweises auf die Patenterteilung:
**09.12.81 Patentblatt 81/49**

(84) Benannte Vertragsstaaten:
**AT BE CH FR GB IT LU NL SE**

(56) Entgegenhaltungen:
**DE-A-2 504 343**
**FR-A-1 549 418**
**FR-A-2 067 677**
**US-A-2 403 272**
**US-A-3 256 932**

(73) Patentinhaber: **Linde Aktiengesellschaft,**
**Abraham-Lincoln-Strasse 21, D-6200 Wiesbaden (DE)**

(72) Erfinder: **Bräutigam, Max, Robert-Koch-Strasse 9,**
**D-8012 Ottobrunn (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

Wärmetauscher für Hochdruck- und Hochtemperatureinsatz und Verfahren zu seiner Herstellung sowie Verwendung als Reaktor

Die Erfindung betrifft einen Wärmetauscher für zwei Fluide mit in einem geschlossenen zylindrischen Behälter verlaufenden Rohre zur Führung des ersten Fluids, bei dem der Aussenraum um die Rohre zur Aufnahme des zweiten Fluids vorgesehen ist und bei dem alle Zuführungen und Abführungen im Deckel des Behälters angeordnet sind, wobei die Rohre vom Deckel aus in der Nähe der Behälterwand in Richtung Behälterboden, von dort zur Behälterachse hin und innerhalb der äusseren Rohrabschnitte zum Deckel zurückverlaufen und an beiden Enden in den durch den Deckel nach aussen geführte Sammelrohre münden, und wobei die äusseren und inneren Abschnitte der Rohre durch einen Mantel voneinander getrennt sind, der an seiner Unterseite gegen den Behälterinnenraum offen ist, sowie ein Verfahren zur Herstellung eines derartigen Wärmetauschers und seine Verwendung als Reaktor.

Ein Wärmetauscher dieser Art ist durch die US-A-2 403 272 bekanntgeworden. Er kann jedoch, insbesondere wenn Fluide mit hohem Druck und hoher Temperatur miteinander in Wärmetausch gebracht werden sollen, nur begrenzt eingesetzt werden. In solchen Anwendungsfällen ergeben sich bei dem beschriebenen Wärmetauscher erhebliche Probleme. Zum einen erfordern Betriebstemperaturen in der Grössenordnung von 900 bis 1300 K den Einsatz hochwertiger Speziallegierungen, die wegen ihrer Legierungsbestandteile teuer sind und in diesem Temperaturbereich bereits geringe Festigkeit aufweisen. Zum anderen müssen, wenn die Fluide auch noch unter Druck vorliegen, die Wandstärken der druckbelasteten Teile vergrössert werden, was die Kosten weiter erhöht. Ausserdem werden bei grösserer Dimensionierung des Wärmetauschers sehr bald Abmessungen erreicht, bei denen die Belastbarkeit des Wärmetauschers nicht mehr nach Standardtabellen berechenbar und das Material nicht mehr beschaffbar ist. Auch nimmt der Wärmetauscher ein derartiges Gewicht an, dass er nicht mehr transportierbar ist.

Der Erfindung liegt daher die Aufgabe zugrunde, einen Wärmetauscher zu entwickeln, der auch noch bei hohen Betriebstemperaturen und Betriebsdrücken einsetzbar ist und der kostengünstig hergestellt werden kann.

Diese Aufgabe wird erfindungsgemäss dadurch gelöst, dass der Mantel an seiner Oberseite dicht mit einem konzentrisch um das innere Sammelrohr angeordneten zweiten Rohr zur Durchführung des zweiten Fluids durch den Deckel verbunden ist und dass konzentrisch unter Bildung von Ringräumen um die äusseren Sammelrohre für das erste Fluid und/oder um das zweite Rohr jeweils weitere Rohre angeordnet sind, die mit dem ringförmigen Teilraum zwischen Mantel und Behälterwand in Verbindung stehen und teilweise als Durchführungen für das zweite Fluid ausgebildet sind.

Das Innere des Wärmetauschers ist durch einen zylindrischen Mantel in einen inneren Teilraum mit kreisförmigem Querschnitt und in einen äusseren, um den inneren Teilraum angeordneten ringförmigen Teilraum, der sich zwischen Mantel und Gehäusewand erstreckt, aufgeteilt. Die beiden Teilräume stehen nur in der Nähe des Behälterbodens miteinander in Verbindung. Erfindungsgemäss ist der Mantel dicht mit einem Rohr verbunden, das zentrisch durch den Deckel aus dem Behälter geführt und als Durchführung für das zweite Fluid ausgebildet ist. Entlang der Teilräume verlaufen Rohre für das erste Fluid. Die Rohrabschnitte im inneren Teilraum münden in ein innerhalb des mit dem Mantel verbundenen Rohres angeordnetes Sammelrohr, die äusseren Rohrabschnitte münden in mehrere über dem äusseren Teilraum befindliche Sammelrohre, wobei die Sammelrohre zur Zu- und Abführung des ersten Fluids dienen. Um das zentrische Zuführungsrohr für das zweite Fluid und um die äusseren Sammelrohre für das erste Fluid sind unter Bildung von Ringräumen mit dem Deckel verbundene weitere Rohre angeordnet, wobei die Ringräume zwischen den Sammelrohren und den weiteren Rohren mit dem äusseren Teilraum in Verbindung stehen und die um die äusseren Sammelrohre angeordneten weiteren Rohre als Durchführungen für das zweite Fluid ausgebildet sind. Die Fluide werden in der Weise in den Wärmetauscher geführt, dass sich das heisse Ende in der Achse des Wärmetauschers befindet und das kalte Ende des Wärmetauschers ausserhalb der Achse. Sämtliche Zylinderwände des Wärmetauschbehälters, der Boden, die äusseren Durchführungen und Sammelrohre und der äussere Teil des Deckels befinden sich dann beim erfindungsgemässen Wärmetauscher auf der kalten Seite. Die einzigen Bauteile des Wärmetauschers, die hohen Temperaturen ausgesetzt sind, sind das zentrische Sammelrohr für das erste Fluid, die mit dem Mantel verbundene Durchführung für das zweite Fluid und das um diese Durchführung angeordnete weitere Rohr, das jedoch an seinem unteren Ende mit der kalten Seite des Deckels verbunden ist und daher nur im oberen Teil kritische Temperaturen erreicht. Sämtliche heissen und damit kritischen Bauteile weisen beim erfindungsgemässen Wärmetauscher kleine Durchmesser auf.

Mit der erfindungsgemässen Konstruktion eines Wärmetauschers wird der Vorteil erreicht, dass nicht der gesamte Wärmetauscher hohen Temperaturen ausgesetzt ist, sondern nur noch Teilbereiche mit einfacher Geometrie, die auch bei grosser Auslegung des Wärmetauschers klein dimensioniert werden können. Der erfindungsgemässe Wärmetauscher kann daher auch bei hohen Temperaturen und Drücken eingesetzt und mit grossen Abmessungen geliefert werden. Die im kritischen Bereich auftretenden Störstellen im Spannungsverlauf sind klein, und die dort auftretenden Spannungen lassen sich mit Standardrechnungen bestimmen.

Gemäss einer vorteilhaften Ausgestaltung des Erfindungsgegenstandes ist das um das zweite Rohr angeordnete weitere Rohr mit einer Kühlentwicklung versehen. Das weitere Rohr besitzt an seinem oberen Ende im wesentlichen die Temperatur des heisseren der beiden Fluide, mit dessen Durchführung es hier verbunden ist, während es am unteren Ende am Deckel des Wärmetauschers befestigt ist und dort die Temperatur des kälteren Fluids besitzt. Da in dem Ringraum zwischen dem zweiten Rohre und dem um das zweite Rohr angeordneten weiteren Rohr keine Strömung herrscht, wird ein gewisser Anteil der Wärme im zweiten Rohr auf das weitere Rohr übertragen. Die Kühlwicklung dient zur Abführung der Wärmemenge, die einerseits durch Wärmeleitung und andererseits durch Strahlung und Konvektion vom zweiten Rohr her auf das weitere Rohr übertragen wird.

Es erweist sich als günstig, wenn gemäss einer weiteren Ausgestaltung des erfindungsgemässen Wärmetauschers mit Abstand zum Deckel des Wärmetauschbehälters eine mit dem Deckel fest verbundene Versteifungsplatte angeordnet ist. Die Versteifungsplatte kann sowohl innerhalb als auch ausserhalb des Wärmetauschbehälters angebracht sein. Ist der Deckel des Wärmetauschers eben, so empfiehlt sich die Verwendung einer ebenen Versteifungsplatte, während bei einem gekrümmten Deckel die Versteifungsplatte sowohl eben als auch gekrümmt sein kann. Die Verbindung zwischen Deckel und Versteifungsplatte kann sowohl über die weiteren Rohre und/ oder die Seitenwand des Behälters und/oder über sonstige Zuganker erfolgen. Die Versteifungsplatte nimmt einen Teil der Druckkräfte auf, denen der Deckel ausgesetzt ist. Die Wandstärke des Deckels kann bei gleichem Druckaufnahmeverfahren verringert werden. Dieser Vorteil bewirkt niedrigere Materialkosten.

Bei einer bevorzugten Ausführungsform des Gegenstandes der vorliegenden Erfindung sind die inneren Rohrabschnitte mit einem Dichthemd versehen, das mit dem Mantel verbunden ist.

Das Dichthemd hat den Zweck, den Raum zwischen den Rohren und dem Mantel abzudichten, um ein Hinterströmen der Rohre durch das im Aussenraum geführte Fluid und damit eine Verschlechterung der Wärmeübertragung zu verhindern. Zweckmässigerweise ist zwischen Mantel und Dichthemd eine Wärmeisolierung vorgesehen.

Bei einer weiteren bevorzugten Ausführungsform des Erfindungsgegenstandes sind die äusseren Rohrabschnitte mit einem Dichthemd versehen, das mit der Innenseite der Behälterwand verbunden ist. Damit wird ebenfalls ein Hinterströmen der Rohre verhindert. Zur Absenkung der Temperatur an der Behälterwand wird als weitere Ausgestaltung das Ausfüllen des Zwischenraumes zwischen Dichthemd und Behälterwand mit einer Wärmeisolierung vorgeschlagen.

Als vorteilhafte Weiterbildung des Erfindungsgedankens wird vorgeschlagen, den Behälter mindestens teilweise mit einer Schüttung zu füllen. Die Schüttung schützt den Wärmetauscher vor Verunreinigungen, z. B. Staub- oder Russteilchen, die durch das im Aussenraum der Rohre geführte Fluid eingetragen werden.

Sollen in dem Wärmetauscher exotherme Reaktionen durchgeführt werden, so ist es zweckmässig, wenn gemäss einer vorteilhaften Ausführungsform des Erfindungsgegenstandes die Schüttung ein Katalysatormaterial enthält.

Um Verunreinigungen, die sich innerhalb des Mantels zwischen den inneren und den äusseren Rohrabschnitten ansammeln, entfernen zu können, sowie um die Rohrwicklungen zu Inspektionszwecken zugänglich zu machen und zum Auswechseln der Schüttung ist es günstig, wenn bei einem erfindungsgemässen Wärmetauscher, bei dem um die äusseren Sammelrohre für das erste Fluid kein weiteres Rohr angeordnet ist, um das um das zweite Rohr angeordnete weitere Rohr ein mit dem Deckel verbundener Zylinder, der mindestens den Durchmesser der inneren Rohrabschnitte aufweist und an dem Durchführungen für das zweite Fluid vorgesehen sind, angeordnet ist und die mit den äusseren Rohrabschnitten verbundenen Sammelrohre durch den Zylinder nach aussen geführt sind. Die Aufhängung der äusseren Rohrabschnitte kann sowohl am Zylinder als auch an den oberen Enden der äusseren Rohrabschnitte erfolgen.

Dadurch, dass das weitere Rohr denselben Durchmesser wie der Mantel aufweist, kann die gesamte Innenwicklung des Wärmetauschers aus dem Mantel gezogen werden, ohne dass der Deckel des Wärmetauschers beschädigt werden müsste. Bei herausgezogener Innenwicklung lässt sich ausserdem die Schüttung im Raum zwischen Mantel und Behälterwand auswechseln.

Es hat sich gezeigt, dass der beschriebene Wärmetauscher hinsichtlich seiner Einsatzmöglichkeiten beschränkt ist, da zum einen durch die Grösse des Wärmetauschers die Grössenordnung der Menge der wärmetauschenden Fluide festgelegt ist, und zum anderen die Anzahl der verwendeten Fluide wegen der relativ schlechten Zugänglichkeit vor allem der äusseren Rohrwicklungen des Wärmetauschers zu Reinigungszwecken auf «saubere» Fluide beschränkt ist, d. h. auf Fluide, die nur wenig Rückstände im Wärmetauscher hinterlassen. Aus diesem Grund ist bei einer zweckmässigen Weiterbildung des Erfindungsgegenstandes im Behälterboden des Wärmetauschers eine verschliessbare Öffnung vorgesehen.

Durch die Öffnung im Boden kann eine Verbindung zwischen dem Mantelraum des Wärmetauschers und dem Aussenraum hergestellt werden. Da der Behälterboden auf einer erheblich niedrigeren Temperatur als das heisse Ende des Wärmetauschers liegt und in diesem Sinn kein kritisches Bauteil darstellt, lässt sich das Anbringen einer Öffnung in diesem Bereich mit den Sicherheitsanforderungen in Einklang bringen.

Die Öffnung erfüllt zweierlei Funktionen, die sich beide als vorteilhaft erweisen. Erstens können Verunreinigungen, die von dem abzukühlenden Fluid in den Wärmetauscher eingetragen werden und sich dort absetzen oder die bei chemischen Reaktionen im Wärmetauscher entstehen, durch die Bodenöffnung aus dem Wärmetauscher entfernt werden. Zweitens kann die erfindungsgemässe Öffnung dazu benutzt werden, um ein kaltes Fluid zuzuführen oder ein abgekühltes Fluid abzuführen. Bei diesem Betrieb wird die Zuführungs- bzw. Ausführungsöffnung im Deckel des Wärmetauschers verschlossen. Der Strömungsweg des mantelseitigen Fluids wird auf diese Weise um die Hälfte verkürzt und ein Teillastbetrieb des Wärmetauschers ermöglicht.

Um rohrseitig eine optimale Wärmeübertragung zu ermöglichen, ist bei einer vorteilhaften Weiterbildung des Erfindungsgegenstandes zwischen der Behälterwand und den äusseren Rohrabschnitten eine parallel zu den äusseren Rohrabschnitten geschaltete Kühlwicklung vorgesehen und sind die Sammelrohre für die äusseren Rohrabschnitte über eine abschliessbare Kurzschlussleitung mit dem Sammelrohr der inneren Rohrabschnitte verbunden, wobei sowohl die Kühlwicklung als auch die Zuführung zu den äusseren Rohrabschnitten unabhängig voneinander abschliessbar ist. Je nach der Stellung der Absperrventile werden die inneren und die äusseren Rohrabschnitte entweder in derselben oder in umgekehrter Richtung durchflossen, so dass in den äusseren Rohrabschnitten die Strömungsrichtung den jeweiligen Betriebsbedingungen angepasst werden kann.

In vorteilhafter Weise ist bei einer Modifikation des Erfindungsgegenstandes mit Abstand zum Behälterboden eine den Behälterquerschnitt überspannende, mit Gasdurchtrittsöffnungen versehene Platte angebracht.

Bei einer weiteren vorteilhaften Ausführungsform des Erfindungsgegenstandes ist auch die Zuführung für die Kühlwicklung durch den Zylinder aus dem Behälter geführt.

Gemäss einer bevorzugten Modifikation des Erfindungsgegenstandes sind im Bereich des Eintritts und/oder des Austritts des zweiten Fluids innerhalb des Behälters Filter vorgesehen, um Fluide, die Verunreinigungen enthalten, zu reinigen. Da die Filter erfindungsgemäss innerhalb des Wärmetauschers angeordnet sind, können auch Fluide, die Staubteilchen, Russ oder ähnliche Verunreinigungen enthalten, für den Wärmetausch verwendet werden, ohne dass externe Hochtemperatur- und Hochdruckbauteile vonnöten sind.

Um bei der Verwendung von Verunreinigungen enthaltenden Fluiden auf der Mantelseite Ablagerungen von den äusseren Rohrwicklungen entfernen zu können, ist gemäss einer Weiterbildung des Erfindungsgegenstandes der Mantel als Korb ausgebildet. Nach Herausziehen der inneren Rohrwicklung können die Ablagerungen durch die Korböffnungen hindurch abgekratzt werden.

Zur Abdichtung des Mantels und um ein Hinterströmen der Rohrwicklungen zu verhindern, sind an der Innenseite des als Korb ausgebildeten Mantels und/oder an der Aussenseite der Kühlwicklung Dichthemden vorgesehen, die mit dem Mantel bzw. mit der Behälterwand verbunden sind.

Der erfindungsgemässe Wärmetauscher ist besonders zur Verwendung als Reaktor bei der Methansynthese geeignet. Hierbei wird Kohlenmonoxid und Wasserstoff mantelseitig in den Wärmetauscher eingeleitet und reagiert dort unter Anwesenheit nickelhaltiger Katalysatoren in einer stark exothermen Reaktion zu Methan gemäss der Gleichung $CO + 3 H_2 \rightarrow CH_4 + H_2O + 206{,}32 kJ/Mol\ CO$

Zur Herstellung eines erfindungsgemässen Wärmetauschers mit gewickelten inneren und äusseren Rohrabschnitten wird ein Verfahren vorgeschlagen, bei dem in getrennten Arbeitsgängen die Rohrabschnitte auf ein zweites Kernrohr, dessen Innendurchmesser grösser ist als der Durchmesser der äussersten Wicklung der inneren Rohrabschnitte, gewickelt werden, anschliessend die beiden gewickelten Abschnitte ineinander geschoben, die Rohrquerschnitte miteinander verbunden und zuletzt beide Abschnitte mit einem Gehäuse umgeben werden.

Bisher war es üblich, gewickelte Wärmetauscher in einem Arbeitsgang auf ein Kernrohr zu wickeln. Mit dieser Methode werden bei einem Gewicht des Wärmetauschers von etwa 70 t bis 120 t herstellungstechnische Grenzen erreicht. Das Kernrohr, auf das die Wicklungen aufgebracht werden, ist während der Fertigung an seinen beiden Enden drehbar gelagert. Erreicht der Wärmetauscher ein Gewicht von 70 bis 120 t, knickt das Kernrohr in der Mitte durch. Als Abhilfe wäre es denkbar, den Durchmesser des Kernrohres zu vergrössern, damit vergrössert sich aber auch das Schadvolumen des Wärmetauschers. Mit dem erfindungsgemäss vorgeschlagenen Herstellungsverfahren wird ebenfalls das Kernrohr bis zur maximal möglichen Last mit Wicklungen versehen. Da jedoch der äussere Teil der Wicklungen auf ein separates Kernrohr gewickelt wird, kann dieser Teil ebenfalls bis zu einem Gewicht von 70 bis 120 t ausgelegt werden. In der Praxis kann ein grösseres Gewicht erreicht werden, da das äussere Kernrohr einen grösseren Durchmesser aufweist und daher stabiler ist. Werden beide Teile gleichzeitig gefertigt, verkürzt sich die Bauzeit für den Wärmetauscher um die Hälfte. Beim Transport auf die Baustelle erweist es sich wiederum als günstig, dass der erfindungsgemässe Wärmetauscher aus mehreren Teilen gefertigt ist, da die Grenze der Transportierbarkeit mit herkömmlichen Transportmitteln bei etwa 150 t Gewicht liegt. Die Einzelteile des mit dem erfindungsgemässen Verfahren hergestellten Wärmetauschers bleiben deutlich unterhalb dieser Grenze und können daher ohne weiteres transportiert werden. Auf der Baustelle kann aus den Teilen jedoch ein Wärmetauscher von weit über 150 t Gewicht zusammengebaut werden.

Es ist selbstverständlich möglich, dass das erste Fluid aus mehreren verschiedenartigen Bestandteilen besteht, die getrennt voneinander durch die Rohre strömen. In diesem Fall weisen die Durchführungen getrennte Strömungswege für die Bestandteile auf.

Weitere Einzelheiten der vorliegenden Erfindung werden anhand von schematisch dargestellten Ausführungsbeispielen beschrieben.

Hierbei zeigt:
Figur 1 einen erfindungsgemässen Wärmetauscher im Querschnitt,
die Figuren 2a bis e verschiedene Ausführungen und Anordnungen einer Versteifungsplatte für den Deckel eines erfindungsgemässen Wärmetauschers,
Figur 3 eine modifizierte Ausführungsform des erfindungsgemässen Wärmetauschers,
Figur 4 eine weitere Modifikation des erfindungsgemässen Wärmetauschers,
Figur 5 eine weitere Modifikation des erfindungsgemässen Wärmetauschers.

Am Beispiel der Kohlevergasung mit Hilfe eines Druckwasserreaktors soll das Prinzip eines erfindungsgemässen Wärmetauschers gezeigt werden. Im Wärmetauscher wird der Wärmeinhalt des Reaktionsgases auf den im Kernreaktor erzeugten Dampf übertragen. Der Verfahrensdruck ist auf beiden Seiten etwa 60 bar, die Verfahrenstemperatur am warmen Ende liegt bei 900 bis 1250 K. Es ist dabei zu berücksichtigen, dass der Wirkungsgrad der gesamten Anlage bei steigender Temperatur und steigendem Druck auf der Reaktordampfseite zunimmt. Der Reaktordampf liegt z. B. mit einer Temperatur von 500 bis 600 K vor.

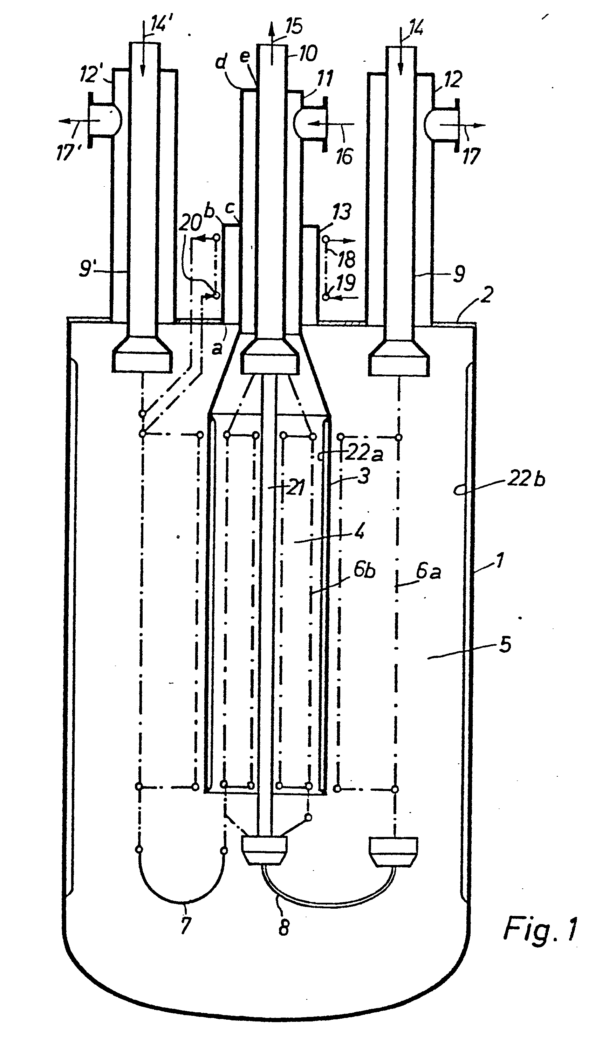

Der Wärmetauscher gemäss Figur 1 besteht aus einem zylindrischen Behälter 1 mit vertikaler Hauptachse, der an seiner Oberseite mit einem Deckel 2 verschlossen ist. Im Behälterinnenraum ist um die Behälterachse ein zylindrischer Mantel 3 angeordnet, der den Behälterinnenraum in einen kreisförmigen inneren Teilraum 4 und einen ringförmigen äusseren Teilraum 5 trennt. Beide Teilräume 4, 5 stehen am unteren Ende des Mantels miteinander in Verbindung. Durch die Teilräume 4, 5 verlaufen Rohre 6a, 6b, die vom Deckel 2 an der Behälteraussenwand entlang in Richtung Boden, dort zur Behälterachse hin und innerhalb des Mantels 3 zum Deckel 2 zurückverlaufen. Die Rohre sind zweckmässigerweise zu Rohrbündeln zusammengefasst und im äusseren Teilraum 5 um den Mantel 3 und im inneren Teilraum 4 um ein Kernrohr 21 gewickelt. Ein gewickelter Wärmetauscher ermöglicht wegen des guten Wärmeübergangs und der grossen Heizflächendichte bei vorgegebener Grösse und vorgegebenem Gewicht den grössten Wärmeumsatz. Die Art der Wicklung richtet sich nach den Anforderungen. Selbstverständlich ist es auch möglich, die Rohre im wesentlichen geradlinig durch die Teilräume 4, 5 zu führen oder gewickelte und geradlinige Abschnitte in den beiden Teilräumen 4, 5 zu kombinieren. Die Rohre können sowohl aus einem Stück gefertigt und durch Umbiegen aus dem Teilraum 5 in den Teilraum 4 geführt sein, wie dies bei 7 angedeutet ist, es ist jedoch auch möglich, die zwei getrennten Rohrabschnitte 6a und 6b über miteinander verbundene Rohrböden zusammenzuführen, wie dies bei 8 angedeutet ist. Die Verbindung 7 oder 8 trägt dazu bei, Spannungen, die aufgrund des Temperaturunterschieds in den Rohrabschnitten 6a und 6b auftreten, auszugleichen. An ihrem oberen Ende münden die äusseren Rohrabschnitte 6a in Sammelrohre 9, 9'. Die Anzahl der Sammelrohre wird den Bertriebsanforderungen angepasst. Die inneren Rohrabschnitte 6b münden in ein zentrisch im Behälter angeordnetes Sammelrohr 10. Das zentrische Sammelrohr 10 ist unter Bildung eines Ringraumes von einem koaxialen zweiten Rohr 11 umgeben, das als Zuführung für das heisse Reaktionsgas ausgebildet ist und das an seiner Unterseite dicht mit dem Mantel 3 verbunden ist. Die Teilräume 4 und 5 sind dadurch in Deckelnähe voneinander getrennt. Koaxial um die Sammelrohre 9 und 9' sind weitere Rohre 12, 12' angeordnet, die als Abführungen für das abgekühlte Reaktionsgas ausgebildet sind und die auf der Unterseite fest mit dem Deckel 2 verbunden sind. Das um das zentrische Sammelrohr 10 angeordnete zweite Rohr 11 ist von einem weiteren Rohr 13 unter Bildung eines mit dem Teilraum 5 verbundenen Ringraums umgeben. Im hier beschriebenen Beispiel wird der Reaktordampf bei 14, 14' mit 500 bis 600 K in die Sammelrohre 9, 9' geleitet und bei 15 mit etwa 850 bis 1200 K aus dem zentrischen Sammelrohr abgeführt. Das heisse Reaktionsgas mit etwa 900 bis 1250 K wird bei 16 in das zentrale zweite Durchführungsrohr 11 geleitet, kühlt sich durch Wärmetausch mit dem Reaktordampf ab und verlässt den Wärmetauscher bei 17, 17' mit etwa 550 bis 700 K. Es ist auch eine andere Führung der Fluide als die beschriebene möglich. So kann das heissere der Fluide durch das Sammelrohr 6 und das kältere über die Durchführungen 12, 12' zugeführt werden. Entscheidend ist, dass das heisse Ende des Wärmetauschers in der Behälterachse liegt. In dem Beispiel ist es sinnvoller, das Reaktionsgas nicht in den Rohren zu führen, weil in dem Reaktionsgas enthaltene Russ- und Staubteilchen an den unzugänglichen Rohrinnenwänden zu unerwünschter Korrosion und Verlegung führen. Um das weitere Rohr 13 ist eine Kühlwicklung 18 vorgesehen, die entweder von einem dritten Fluid (wie bei 19) oder von dem kälteren der beiden wärmetauschenden Fluide (wie bei 20) durchflossen ist. Die inneren und äusseren Abschnitte 6a, 6b der Rohre des Wärmetauschers sind von Dichthemden 22a, 22b umschlossen. Die Dichthemden umschliessen jeweils die äussere Lage der Rohre dicht und sind mit ihren Rändern am Mantel 3 bzw. an der Behälterwand befestigt. Sie verhindern ein Hinterströmen der Rohre durch das Reaktionsgas. Zwischen dem äusseren Dichthemd 22b und der Behälterwand kann eine Wärmeisolierung eingefügt sein, die jedoch nicht dargestellt ist. Die Isolierung kann sich über die gesamte Behälterwand erstrecken. Auch zwischen dem Mantel 3 und dem Dichthemd 22a kann bei Bedarf eine Wärmeisolierung vorgesehen sein. Zum Verständnis der Wirkungsweise des Wärmetauschers sei erwähnt, welche Bauteile kritischen Temperaturen und Drücken ausgesetzt sind. Innerhalb des Behälters 2, den Durchführungen für die Fluide und in den Rohren herrscht überall ein Druck von etwa 60 bar. Die Zuführungen 9, 9', 11, die Abführungen 10, 12, 12', der Behälter 1, der Deckel 2, die weiteren Rohre 13 und aus Sicherheitsgründen, für den Fall, dass das Reaktionsgas ausfällt, üblicherweise auch die Rohre, in denen der Dampf vom Kernreaktor strömt, müssen für diesen Druck ausgelegt sein. Da auf beiden Seiten des Mantels 3 annähernd derselbe Druck herrscht, braucht der Mantel 3 nicht für Hochdruck ausgelegt zu sein.

Die höchsten Temperaturen im Wärmetauscher werden im Sammelrohr 10, im zweiten Rohr 11 und im oberen Teil des weiteren Rohres 13 erreicht. Diese Bauteile sind aus hochwertigen Legierungen, z. B. Incoloy, gefertigt. Durch die Kühlwicklung 18 und den Kontakt mit dem Deckel 2 stellt sich im weiteren Rohr 13 nach unten ein negativer Temperaturgradient ein. Der Ringraum zwischen den Rohren 11 und 13 ist mit Reaktionsgas gefüllt. Durch den Ringraum und die Kühlwicklung ist der Deckel weitgehend von dem heissen Rohr 11 thermisch getrennt. Der Ringraum wird zweckmässigerweise nicht zu dick gebaut, um dort Gasturbulenzen zu vermeiden. Ausserdem kann zur Verbesserung der Wärmeisolierung ein Isoliermaterial in den Ringraum eingebracht sein. Die Störstellen a, b, c, d, e im Spannungsverlauf sind mit in der Figur nicht eingezeichneten Ringversteifungen versehen.

Die besonders korrosionsgefährdeten Teile innerhalb des Mantels 3 sind leicht zugänglich, da auf beiden Seiten des Mantels derselbe Druck herrscht und der Mantel deshalb unbedenklich aufgeschnitten und wieder zugeschweisst werden kann, weil er kein Druckbehälterbauteil darstellt.

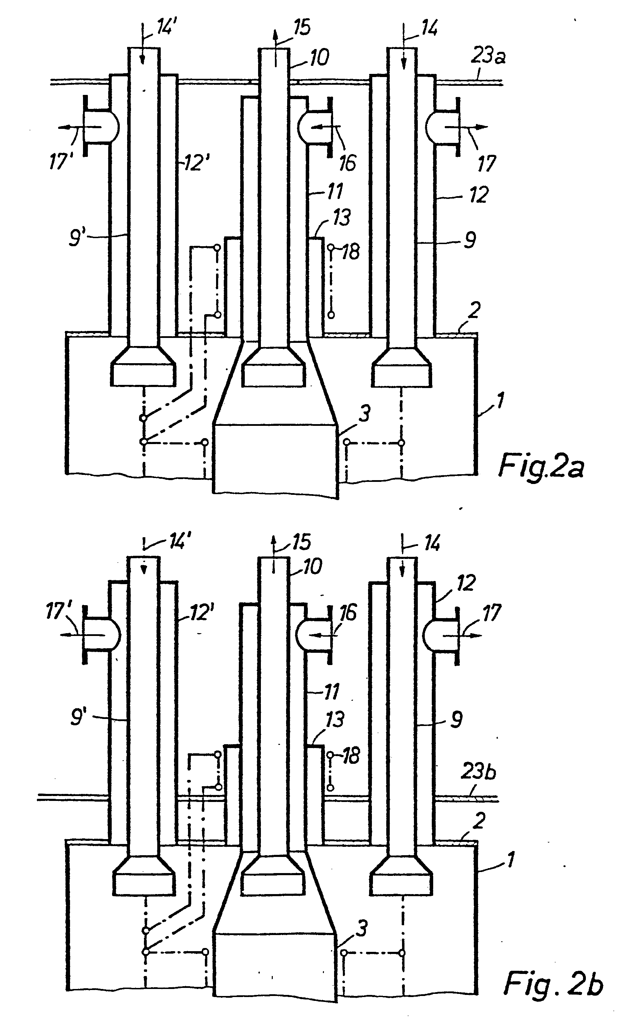

Die Figuren 2a bis e zeigen verschiedene Möglichkeiten, den Deckel 2 des Wärmetauschers mit Hilfe einer Versteifungsplatte 23a bis e zu entlasten. Gleiche Bauteile tragen dieselben Bezugszeichen wie in Fig. 1. In allen Beispielen hat die Versteifungsplatte etwa dieselbe Temperatur wie die Fluide auf der kalten Seite des Wärmetauschers. In den Figuren 2a und 2b ist die Kombination des ebenen Deckels 2 mit ebenen Versteifungsplatten 23a und 23b dargestellt. Die Platte 23a in Figur 2a ist mit den weiteren Rohren 12, 12' verbunden und weist eine Aussparung für das heisse Sammelrohr 10 auf. In Figur 2b ist die Versteifungsplatte 23b unterhalb der Kühlwicklung 18 angeordnet und ausser an den weiteren Rohren 12, 12' auch am weiteren Rohr 13 befestigt. Durch die zusätzliche Befestigung in der Mitte hat die Biegelinie für den Deckel 2 bei Belastung einen weitaus günstigeren Verlauf.

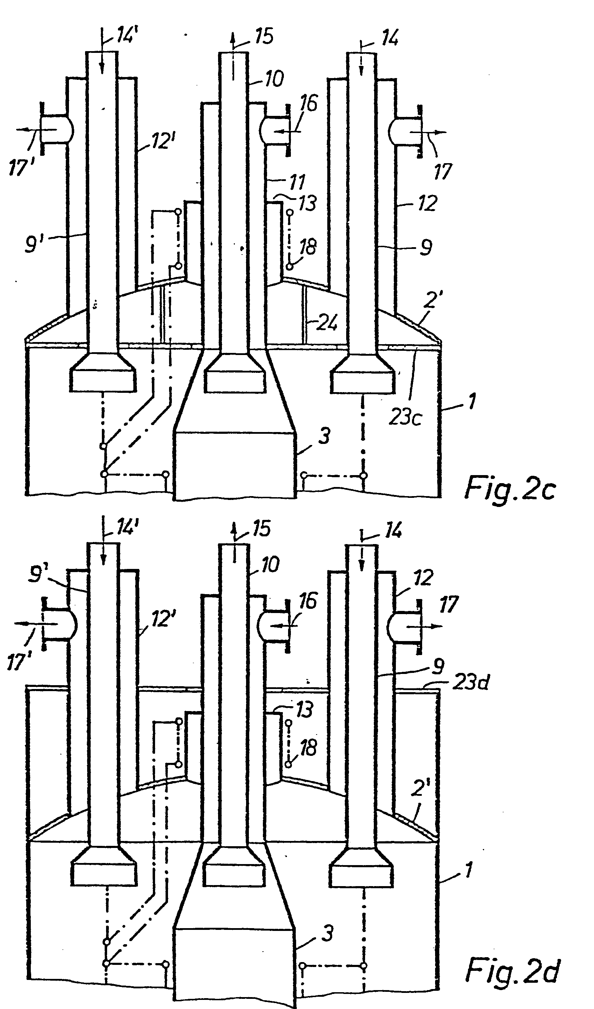

In Figuren 2c und 2d zeigen mögliche Kombinationen eines gewölbten Deckels 2' mit ebenen Versteifungsplatten 23c und 23d. Die Versteifungsplatte 23c in Figur 2c ist innerhalb des Behälters 1 angeordnet und an der Behälterwand sowie über Zuganker 24 am Deckel 2' befestigt. Für die Sammelrohre 9, 9' und das weitere Rohr 11 sind Aussparungen vorgesehen, durch die das Reaktionsgas strömt. Gemäss einer weiteren Ausführungsform des erfindungsgemässen Wärmetauschers ist die Behälterwand 2 bis zu der oberhalb des Deckels angeordneten Versteifungsplatte 23d hochgezogen und mit dieser verschweisst (Figur 2d). Die Versteifungsplatte 23d ist ausserdem mit den weiteren Rohren 12, 12' verschweisst. In der Mitte ist eine Durchführung für das zweite Rohr 11 vorgesehen. Selbstverständlich sind neben den gezeigten Anordnungen für die Versteifungsplatte weitere Anordnungen möglich, so kann beispielsweise in Figur 2a oder 2b ebenfalls die Behälterwand bis zur Versteifungsplatte hochgezogen und mit ihr verschweisst sein.

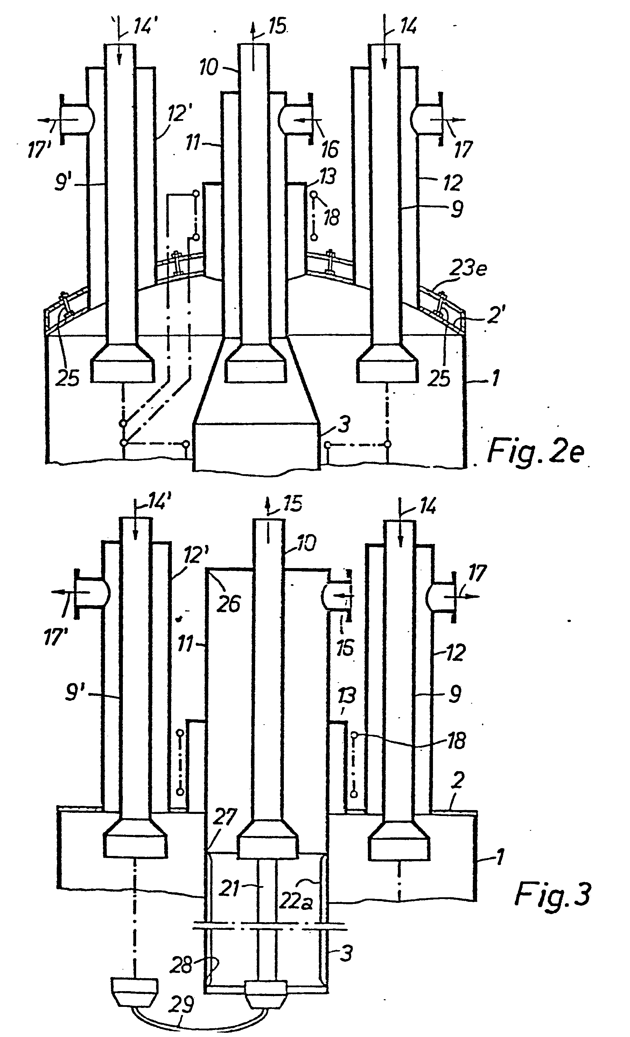

Im Beispiel gemäss Figur 2e ist eine gewölbte Versteifungsplatte mit einem Deckel 2' mit gleicher Wölbung verbunden. Die Verbindung wird über Bolzen 25 hergestellt.

Figur 3 zeigt eine weitere Ausführungsform des erfindungsgemässen Wärmetauschers. Dabei weist das zweite Rohr 11 denselben Durchmesser auf wie der Mantel 3. Die besonders der Korrosion ausgesetzten inneren Rohrabschnitte können mit dem Sammelrohr 10 und dem Dichthemd herausgezogen und gereinigt oder ausgetauscht werden. Hierzu ist es lediglich erforderlich, die Verschlussplatte des zweiten Rohres 11 abzunehmen, sowie die Verbindung des Dichthemds 22a mit dem Mantel 3 zu lösen und die Verbindung zwischen den inneren und den äusseren Rohrabschnitten zu durchtrennen (Schweissnähte 26–29).

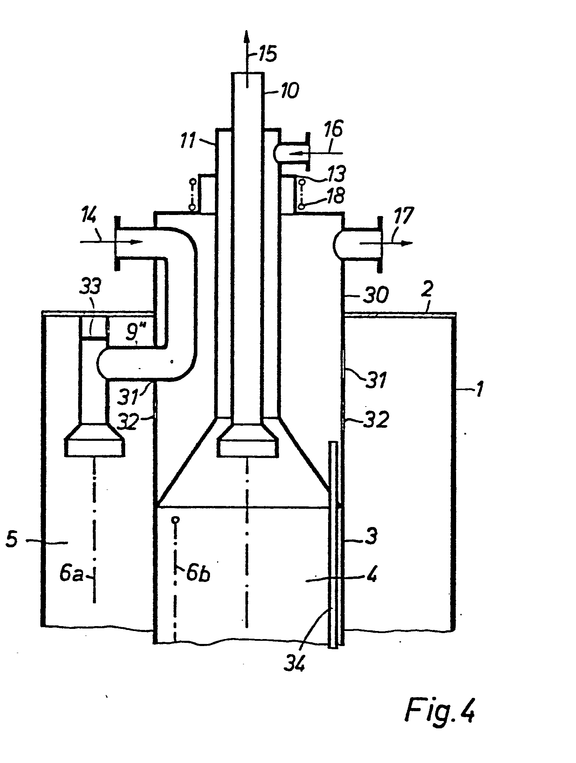

Besonders vorteilhaft ist es (Fig. 4), wenn gemäss einer Modifikation des Erfindungsgegenstandes auf den Behälter 1 ein Zylinder 30 aufgesetzt ist, der das weitere Rohr 13 unterhalb der Kühlwicklung 18 umschliesst und der mindestens den Durchmesser der äussersten Wicklung der inneren Rohrabschnitte 6b aufweist. Bei dieser Ausführungsform kann der Durchmesser des weiteren Rohres 13, das sich an seinem oberen Ende auf hoher Temperatur befindet, klein gehalten werden und trotzdem ein Auswechseln der inneren Rohrabschnitte 6b durchgeführt werden.

Der Zylinder 30 ist bis zum Mantel 3 nach innen verlängert und kann mit diesem als Aufhängevorrichtung für die äusseren Rohrabschnitte 6a verbunden werden. Im Abschnitt zwischen dem Zylinder und dem Mantel 3 sind Öffnungen 31, 32 zur Durchführung der Fluide vorgesehen. Die Öffnungen 31, 32 können ausserdem bei Wartungsarbeiten als Durchstiege benutzt werden.

Der Zylinder 30 nimmt sowohl die Sammelrohre 9'' für den Reaktordampf als auch die Stutzen für den Reaktionsgasaustritt auf, dadurch treten am Behälterdeckel 2 keine zusätzlichen Störstellen auf.

Es ist grundsätzlich auch möglich, die äusseren Rohrabschnitte 6a mit ihren Sammelrohren 9″ am Behälterdeckel 2 aufzuhängen. Die Befestigung ist derart, dass die Sammelrohre T-Stücke aufweisen, deren gerader Durchgang bis zum Deckel 2 geführt und dort als Aufhängung ausgebildet ist. Die Fluidführung geschieht über die Abzweigung. Zum Schutz des Deckels 2 vor zusätzlicher Belastung (Druck, Temperatur) ist über dem T-Stück in der Aufhängung eine Trennplatte 33 eingeschweisst. Der sich darüber ergebende Raum ist über eine Druckausgleichsbohrung mit dem äusseren Teilraum 5 verbunden. Der Deckel 2 hat somit über die gesamte Fläche den gleichen Druck. Sollte jedoch der Druck im Rohrraum eine Entlastung des Deckels 2 bewirken, so ist die Trennplatte und die Ausgleichsbohrung zu unterlassen.

Das Reaktionsgas wird bei 16 eingeleitet und verlässt den Wärmetauscher über am Zylinder 30 angeordnete Stutzen (Pfeil 17).

Bei allen Ausführungsformen des erfindungsgemässen Wärmetauschers ist es möglich, in den Behälter 1 eine Schüttung, z. B. aus einem Katalysatormaterial, einzufüllen.

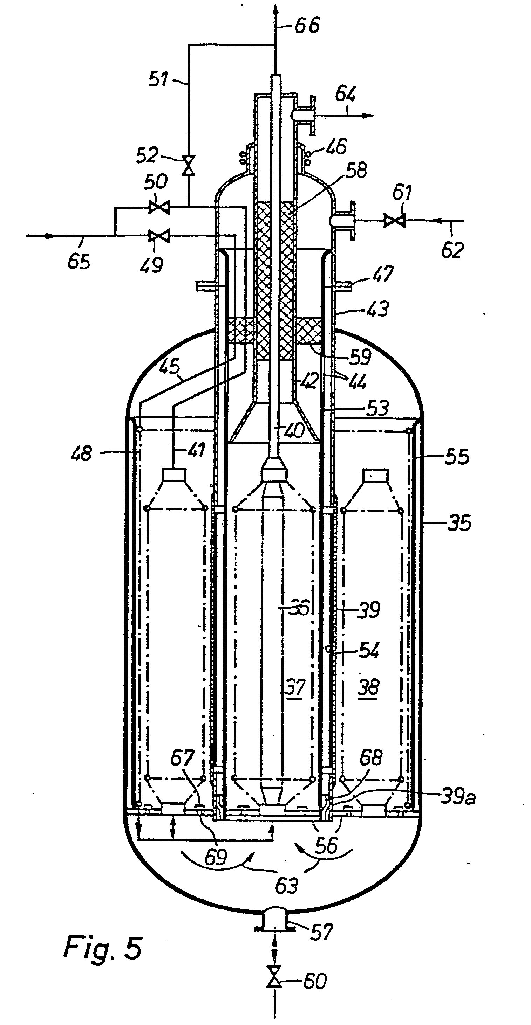

Neben dem genannten Anwendungsbeispiel ist selbstverständlich eine Verwendung des erfindungsgemässen Wärmetauschers bei jedem Wärmetauschvorgang möglich, bei dem die Fluide unter hohem Druck und bei hohen Temperaturen vorliegen. Im folgenden wird ein erfindungsgemässer Wärmetauscher am Beispiel der Methansynthese aus Kohlenmonoxid und Wasserstoff beschrieben. Figur 5 zeigt einen zylindrischen Behälter 35, in dem sich zwei konzentrische Rohrwicklungen befinden, wobei die inneren Abschnitte 37 der Rohre um ein Kernrohr 36, die äusseren Abschnitte 38 der Rohre um einen Mantel 39 gewickelt sind. Der Mantel 39 ist zweckmässigerweise als Korb ausgebildet, der auf einem Stützring 39a befestigt ist, um eine Zugangsmöglichkeit zu den äusseren Rohrabschnitten 38 zu schaffen. Die Rohrabschnitte 37, 38 sind an ihrer Unterseite zu einem fortlaufenden Strömungsweg verbunden. Die freien Enden der Wicklungen münden in Sammelrohre 40, 41, die aus dem Behälter 35 geführt sind. Der Raum innerhalb des Mantels 39 steht über ein konzentrisch zum inneren Sammelrohr 40 angeordnetes zweites Rohr 42, das dicht mit dem Mantel 39 verbunden ist und einen kleineren Durchmesser als dieser aufweist, mit dem Aussenraum in Verbindung. Der Ringraum zwischen Mantel 39 und Behälterwand ist über ein weiteres Rohr 43 mit dem Aussenraum verbunden. Die Verbindung wird durch Öffnungen 44 hergestellt. Wie in der linken Hälfte von Figur 5 schematisch dargestellt ist, dienen die Öffnungen 44 gleichzeitig zur Durchführung der Sammelrohre 41 sowie der Speiserohre 45 für die weiter unten beschriebene Kühlwicklung 48. Grundsätzlich ist es auch möglich, die Rohre 41 und 45 ausserhalb des weiteren Rohrs 43 aus dem Behälter zu führen. Aus Sicherheitsgründen wegen der im Wärmetauscher herrschenden Temperaturen und Drücke werden jedoch unnötige Schweissnähte nach Möglichkeit vermieden. Da das weitere Rohr 43 im wesentlichen denselben Durchmesser aufweist wie der Mantel 39, kann die gesamte innere Rohrwicklung zur Revision o.ä. durch Lösen der Flanschverbindung 47 aus dem Behälter 35 gezogen werden.

Der Übergang vom weiteren Rohr 43 zum heissen Ende des Wärmetauschers ist gekühlt, um die Wärmeleitung in diesem Bereich zu reduzieren (Kühlwicklung 46). Zwischen der äusseren Wicklung und der Behälterwand ist eine Kühlwicklung 48 vorgesehen, die parallel zur äusseren Wicklung geschaltet ist. Mit Absperrventilen 49 und 50 sind die Zuführung zur Kühlwicklung 48 und die Zuführung zur äusseren Rohrwicklung unabhängig voneinander absperrbar. Eine Bypassleitung 51 mit einem Absperrventil 52 verbindet die Zuführleitung zu den äusseren Rohrabschnitten 38 mit der Abführleitung der inneren Rohrabschnitte 37. Die inneren Rohrabschnitte sind mit einem Dichthemd 53 umgeben, das ein Hinterströmen der Wicklung verhindert. Der Raum innerhalb des als Korb ausgebildeten Mantels 39 ist durch ein Dichthemd 54 vom Ringraum zwischen Mantel 39 und Behälterwand abgedichtet. Zwischen der Kühlwicklung 48 und der Behälterwand ist ebenfalls ein Dichthemd 55 vorgesehen. Eine Dichtlippe 68 dichtet den Ringspalt zwischen Stützring 39a und Dichthemd 53 ab.

Mit Abstand zum Behälterboden ist eine Platte 56 angeordnet, die mit Gasdurchtrittsöffnungen 69 versehen ist, von denen in der Figur nur eine dargestellt ist. Die Platte 56 dient als Stützkonstruktion für eine in den Wärmetauscher eingefüllte Katalysatorschüttung. Für die Methanisierung wird zweckmässigerweise ein Nickelgranulat als Katalysator verwendet. Um ein Durchfallen des Katalysators durch die Gasdurchtrittsöffnungen zu verhindern, sind die Öffnungen mit einem engmaschigen Sieb 67 verschlossen.

Je nach den Betriebsbedingungen sind im Ausström- und/oder Einströmquerschnitt des mantelseitigen Fluids Filter 58, 59 angeordnet, um Verunreinigungen zurückzuhalten.

Erfindungsgemäss ist im Boden des Wärmetauschers eine verschliessbare Öffnung 57 vorgesehen. Die Öffnung 57 erfüllt zum einen den Zweck, dass Verunreinigungen, die unterhalb der Platte 56 am Boden des Behälters 35 ausgeschieden werden, bei Bedarf durch Öffnen des Ventils 60 entfernt werden können; zum anderen ermöglicht die Öffnung 57 einen Teillastbetrieb des Wärmetauschers.

Bei Vollastbetrieb ist die Öffnung 57 mit Ventil 60 verschlossen, während die Einlassöffnung für das mantelseitige Fluid geöffnet ist (Ventil 61). Über Leitung 62 wird dem Wärmetauscher Kohlenmonoxid und Wasserstoff zugeführt, im Filter 59 gereinigt und gelangt über die Öffnungen 44 in den Ringraum zwischen Mantel 39 und Behälteraussenwand. Unter Anwesenheit der Katalysatorschüttung entsteht Methan und Wasser. Die Reaktionswärme wird von dem in den Rohren fliessenden Kühlfluid, z. B. Wasser, das über Leitung 65 zugeführt wird, aufgenommen. Das Methan strömt durch die Platte 56, wie durch die Pfeile 63 schematisch angedeutet ist, in den innerhalb des Mantels 39 gelegenen Raum, wo noch nicht umgesetztes Kohlenmonoxid und Wasserstoff zu Methan reagieren, und velässt den Wärmetauscher schliesslich, nachdem durch die innere Rohrwicklung 37 weitere Wäme abgeführt wurde, über das Sammelrohr 42 (Pfeil 66). Im Methan enthaltene Verunreinigungen werden vom Filter 58 zurückgehalten.

Bei Teillastbetrieb des Wärmetauschers ist Ventil 61 geschlossen und Ventil 60 geöffnet. Das mantelseitige Fluid wird wird dem Wärmetauscher über Stutzen 57 zugeführt und strömt nur innerhalb des Mantels 39 nach oben. Auf diese Weise ist sein Strömungsweg um die Hälfte verkürzt.

Grundsätzlich ist sowohl bei Teillast- als auch bei Vollastbetrieb eine umgekehrte Strömungsrichtung für das mantelseitige Fluid möglich. Eine solche wird immer dann gewählt werden, wenn das mantelseitige Fluid dem Wärmetauscher in heissem Zustand zugeführt wird. Um die konstruktiven Vorteile des erfindungsgemässen Wärmetauschers voll ausnützen zu können, muss darauf geachtet werden, dass sich das heisse Ende des Wärmetauschers bei 64 befindet.

Das rohrseitig geführte Kühlfluid kann, um eine den jeweiligen Betriebsbedingungen optimal angepasste Wärmeübertragung zu gewährleisten, je nach der Stellung der Ventile 49, 50 und 52 in der äusseren Wicklung 38 von oben nach unten oder von unten nach oben geführt sein. Im einen Fall ist Ventil 50 geschlossen, die Ventile 49 und 52 sind geöffnet. Das Kühlfluid strömt dann über die Kühlwicklung 48 nach unten und in der inneren und äusseren Wicklung 37, 38 parallel nach oben, wobei das in der äusseren Wicklung 38 strömende Fluid dem in der inneren Wicklung 37 strömenden über Leitung 51 wieder zugeführt wird. Im anderen Fall ist Ventil 52 geschlossen und die Ventile 49 und 50 sind geöffnet. Jetzt strömt das Kühlfluid in der äusseren Wicklung 38 nach unten und in der inneren Wicklung 37 nach oben.

Im erfindungsgemässen Wärmetauscher herrschen bei der isothermen Methansynthese (isotherm bezieht sich auf das Verdampfen des Kühlwassers) folgende Betriebstemperaturen und -drücke:

| | |
|---|---|
| Druck des mantelseitigen Gases | 30 bar |
| Temperatur des mantelseitigen Gases | 800 K |
| Druck des rohrseitigen Wasserdampfes | 100 bar |
| Temperatur des rohrseitigen Wasserdampfes | 600K |

Zuletzt sei noch der Zusammenbau eines erfindungsgemässen Wärmetauschers beschrieben. Der Wärmetauscher wird im wesentlichen aus vier Teilen – innere Wicklung, äussere Wicklung, zylindrischer Behälter mit Deckel, Behälterboden mit einer Standzarge – gleichzeitig gefertigt. Jedes Bauteil für sich kann dabei bis zum maximalen Transportgewicht annehmen.

Am Einsatzort des Wärmetauschers wird zunächst der untere Boden mit Standzarge aufgestellt. In diesem wird ein Säulentisch als Montagehilfe errichtet. Auf dem Montagetisch wird die innere Wicklung mit Dichthemd gestellt. Anschliessend wird die äussere Wicklung, die ebenfalls mit einem Dichthemd geliefert wird, über die innere Wicklung geschoben, wobei das Kernrohr der äusseren Wicklung den Trennmantel zwischen den beiden Wicklungen bildet. Nun wird die Behälterwand mit Deckel und Durchführungen über die äussere Wicklung gesetzt und mit dem unteren Boden verschweisst. Die innere, die äussere Wicklung und der Trennmantel werden mit dem Deckel verbunden, dann der Montagetisch abgebaut. Zuletzt wird das innere Dichthemd mit dem Mantel und das äussere mit der Behälterwand verbunden, ausserdem werden die beiden Wicklungen im unteren Teil des Wärmetauschers verbunden.

Wenn das zur Aufstellung des Montagetisches erforderliche Loch im Behälterboden vermieden werden soll, wird der Wärmetauscher zweckmässigerweise wie folgt zusammengebaut: Zuerst wird der Mantel mit verschweisstem Boden aufgestellt, gleichzeitig werden die innere und die äusseren Wicklungen ineinandergeschoben und miteinander verbunden. In den Wicklungen ist ein parallel zum Kernrohr verlaufendes Rohr 34 (Fig. 4) angeordnet, das vom oberen bis zum unteren Ende der Wicklungen reicht. In den Behälterböden wird Wasser eingefüllt und gefroren. Die Wicklungen werden in den Behälter 1 gehoben und auf der Eisfläche abgestellt. Anschliessend wird der Deckel 2 aufgesetzt und die Verbindungen zwischen den Rohren und den Durchführungen hergestellt. Die Wicklungen hängen nun am Deckel. Das Wasser im Boden wird geschmolzen und über einen durch das Rohr 34 eingeführten Schlauch abgesaugt. Zuletzt wird das Rohr 34 verschlossen.

**Patentansprüche**

1. Wärmetauscher für zwei Fluide mit in einem geschlossenen zylindrischen Behälter (1) verlaufenden Rohren zur Führung des ersten Fluids, bei dem der Aussenraum um die Rohre zur Aufnahme des zweiten Fluids vorgesehen ist, und bei dem die Zuführungen und Abführungen im Deckel (2) des Behälters angeordnet sind, wobei die Rohre vom Deckel (2) aus in der Nähe der Behälterwand in Richtung Behälterboden, von dort zur Behälterachse hin und innerhalb der äusseren Rohrabschnitte (6a, 38) zum Deckel (2) zurückverlaufen und an beiden Enden in durch den Deckel (2) nach aussen geführte innere und äussere Sammelrohre (9, 9′, 10, 40, 41) münden, und wobei die äusseren und inneren Abschnitte (6a, 6b, 37, 38) der Rohre durch einen Mantel (3, 39) voneinander getrennt sind, der an seiner Unterseite gegen den Behälterinnenraum offen ist, dadurch gekennzeichnet, dass der Mantel (3, 39) an seiner Oberseite dicht mit einem konzentrisch um das innere Sammelrohr (10, 36) angeordneten zweiten Rohr (11, 42) zur Durchführung des zweiten Fluids durch den Deckel (2) verbunden ist und dass konzentrisch unter Bildung von Ringräumen um die äusseren Sammelrohre (9, 9', 41) für das 'erste Fluid und/oder um das zweite Rohr (11, 42) jeweils weitere Rohre (12, 12', 13) angeordnet sind, die mit dem ringförmigen Teilraum (5) zwischen Mantel (3, 39) und Behälterwand in Verbindung stehen und teilweise als Durchführungen (12, 12') für das zweite Fluid ausgebildet sind.

2. Wärmetauscher nach Anspruch 1, dadurch gekennzeichnet, dass das um das konzentrisch um das innere Sammelrohr (10) liegende zweite Rohr (11) angeordnete weitere Rohr (13) mit einer Kühlwicklung (18, 46) versehen ist.

3. Wärmetauscher nach Anspruch 1 oder 2, dadurch gekennzeichnet, dass mit Abstand zum Deckel (2) eine mit dem Deckel fest verbundene Versteifungsplatte (23a, 23b, 23c, 23d, 23e) angeordnet ist.

4. Wärmetauscher nach einem der Ansprüche 1 bis 3, dadurch gekennzeichnet, dass die inneren Rohrabschnitte (6b, 37) mit einem Dichthemd (22a, 53) versehen sind, das mit dem Mantel (3, 39) verbunden ist.

5. Wärmetauscher nach einem der Ansprüche 1 bis 4, dadurch gekennzeichnet, dass die äusseren Rohrabschnitte (6a) mit einem Dichthemd (22b) versehen sind, das mit der Innenseite der Behälterwand verbunden ist.

6. Wärmetauscher nach Anspruch 5, dadurch gekennzeichnet, dass zwischen Dichthemd (22b) und Behälterwand eine Wärmeisolierung vorgesehen ist.

7. Wärmetauscher nach einem der Ansprüche 1 bis 6, dadurch gekennzeichnet, dass der Behälter (1, 35) mindestens teilweise mit einer Schüttung gefüllt ist.

8. Wärmetauscher nach Anspruch 7, dadurch gekennzeichnet, dass die Schüttung ein Katalysatormaterial enthält.

9. Wärmetauscher nach einem der Ansprüche 1 bis 8, bei dem um die äusseren Sammelrohre (9, 9') für das erste Fluid keine weiteren Rohre angeordnet sind, dadurch gekennzeichnet, dass um das weitere Rohr (13) ein mit dem Deckel (2) verbundener Zylinder (30, 43), der mindestens den Durchmesser der inneren Rohrabschnitte (6b, 37) aufweist und an dem Durchführungen für das zweite Fluid vorgesehen sind, angeordnet ist, und dass die mit den äusseren Rohrabschnitten verbundenen Sammelrohre (9'', 41) durch den Zylinder (30, 43) nach aussen geführt sind.

10. Wärmetauscher nach Anspruch 9, dadurch gekennzeichnet, dass der Zylinder (30, 43) als Aufhängung für die äusseren Rohrabschnitte (6a, 38) ausgebildet ist.

11. Wärmetauscher nach Anspruch 9, dadurch gekennzeichnet, dass die oberen Enden der äusseren Rohrabschnitte (6a, 38) als Aufhängung ausgebildet sind.

12. Wärmetauscher nach einem der Ansprüche 9 bis 11, dadurch gekennzeichnet, dass der Behälterboden eine verschliessbare Öffnung (57) aufweist.

13. Wärmetauscher nach einem der Ansprüche 9 bis 12, dadurch gekennzeichnet, dass zwischen der Behälterwand und den äusseren Rohrabschnitten (38) eine parallel zu den äusseren Rohrabschnitten (38) geschaltete Kühlwicklung (48) vorgesehen ist und die Sammelrohre (41) für die äusseren Rohrabschnitte (38) über eine abschliessbare Kurzschlussleitung (51) mit dem Sammelrohr (40) der inneren Rohrabschnitte (37) verbunden sind, wobei sowohl die Kühlwicklung (48) als auch die Zuführung zu den äusseren Rohrabschnitten (38) unabhängig voneinander abschliessbar ist.

14. Wärmetauscher nach einem der Ansprüche 9 bis 13, dadurch gekennzeichnet, dass mit Abstand zum Behälterboden eine den Behälterquerschnitt überspannende, mit Gasdurchtrittsöffnungen (69) versehene Platte (56) angebracht ist.

15. Wärmetauscher nach einem der Ansprüche 9 bis 14, dadurch gekennzeichnet, dass auch eine Zuführung (45) für die Kühlwicklung (48) durch den Zylinder (43) aus dem Behälter (35) geführt ist.

16. Wärmetauscher nach einem der Ansprüche 9 bis 15, dadurch gekennzeichnet, dass am Eintritt und/oder am Austritt des zweiten Fluids innerhalb des Behälters Filter (58, 59) vorgesehen sind.

17. Wärmetauscher nach einem der Ansprüche 1 bis 16, dadurch gekennzeichnet, dass der Mantel (39) als Korb ausgebildet ist.

18. Wärmetauscher nach einem der Ansprüche 9 bis 17, dadurch gekennzeichnet, dass an der Innenseite des als Korb ausgebildeten Mantels (3, 39) und/oder an der Aussenwand der Kühlwicklung (48) Dichthemden (54, 55) vorgesehen sind, die mit dem Mantel (39) bzw. mit der Behälterwand verbunden sind.

19. Verwendung eines Wärmetauschers nach einem der Ansprüche 1 bis 18 als Reaktor bei der Methansynthese.

20. Verfahren zur Herstellung eines Wärmetauschers nach einem der Ansprüche 1 bis 19 mit gewickelten inneren und äusseren Rohrabschnitten (37, 38), dadurch gekennzeichnet, dass in getrennten Arbeitsgängen die inneren Rohrabschnitte (37) auf ein erstes Kernrohr (36) und die äusseren Rohrabschnitte (38) gesondert auf ein zweites Kernrohr (39), dessen Innendurchmesser grösser ist als der Durchmesser der äussersten Wicklung der inneren Rohrabschnitte, gewickelt werden, dass beide gewickelten Abschnitte anschliessend ineinandergeschoben, die Rohrquerschnitte miteinander verbunden und beide Abschnitte zuletzt mit einem Gehäuse umgeben werden.

**Claims**

1. A heat exchanger for two fluids comprising pipes which run in a closed cylindrical container (1) and which serve to convey the first fluid, in which the outer chamber surrounding the pipes serves to accommodate the second fluid, and in which the inlets and outlets are arranged in the cover (2) of the container, wherein the pipes extend from the cover (2) in the vicinity of the container walls to the bottom of the container, from whence they extend to the axis of the container and return to the cover (2) within the outer portions of the pipes (6a, 38), the pipes terminating at both ends in inner and outer collector pipes (9, 9', 10, 40, 41) which lead to the outside through the cover (2), and wherein the outer and inner portions (6a, 6b, 37, 38) of the pipes are separated from one another by a casing (3, 39) which is open on its lower end to the interior of the container, characterised in that, at its upper end to casing (3, 39) is connected in sealed fashion to a second pipe (11, 42) which is arranged concentrically to the inner collector pipe (10, 36) and which serves to convey the second fluid through the cover (2), and that further pipes (12, 12', 13) are arranged concentrically with and so as to form annular chambers around the outer collector pipes (9, 9',41) for the first fluid and/or around the second pipe (11, 42), these further pipes being connected to the annular space (5) lying between the casing (3, 39) and the container walls and serving partially as ducts (12, 12') for the second fluid.

2. A heat exchanger as claimed in Claim 1, characterised in that the further pipe (13) which surrounds the second pipe (11) which is arranged concentrically to the inner collector pipe (10), is provided with a cooling coil (18, 46).

3. A heat exchanger as claimed in Claim 1 or 2, characterised in that a stiffening plate (23a, 23b, 23c, 23d, 23e) which is permanently connected to the cover (2) is arranged at a distance therefrom.

4. A heat exchanger as claimed in one of Claims 1 to 3, characterised in that the inner pipe portions (6b, 37) are provided with a sealing shell (22a, 53) which is connected to the casing (3, 39).

5. A heat exchanger as claimed in one of Claims 1 to 4, characterised in that the outer pipe portions (6a) are provided with a sealing shell (22b) which is connected to the inside of the container walls.

6. A heat exchanger as claimed in Claim 5, characterised in that heat insulation is provided between the sealing shell (22b) and the container walls.

7. A heat exchanger as claimed in one of Claims 1 to 6, characterised in that the container (1, 35) is at least partially filled with a packing.

8. A heat exchanger as claimed in Claim 7, characterised in that the packing contains a catalyst material.

9. A heat exchanger as claimed in one of Claims 1 to 8, wherein no further pipes are arranged around the outer collector pipes (9, 9') for the first fluid, characterised in that around the further pipe (13) there is arranged a cylinder (30, 43) which is connected to the cover (2) and which exhibits at least the diameter of the inner pipe portions (6b, 37) and on which ducts for the second fluid are provided, and that the collector pipes (9", 41) which are connected to the outer pipe portions lead to the outside through the cylinder (30, 43).

10. A heat exchanger as claimed in Claim 9, characterised in that the cylinder (30, 43) forms a suspension means for the outer pipe portions (6a, 38).

11. A heat exchanger as claimed in Claim 9, characterised in that the upper ends of the outer pipe portions (6a, 38) form a suspension means.

12. A heat exchanger as claimed in one of Claims 9 to 11, characterised in that the bottom of the container base is provided with a closable opening (57).

13. A heat exchanger as claimed in one of Claims 9 to 12, characterised in that, between the container walls and the outer pipe portions (38), a cooling coil (48) is arranged parallel to the outer pipe portions (38), and the collector pipes (41) for the outer pipe portions (38) are connected via a closable shortcircuit line (51) to the collector pipe (40) of the inner pipe portions (37), wherein both the cooling coil (48) and the supply line to the outer pipe sections (38) can be shut off independently of each other.

14. A heat exchanger as claimed in one of Claims 9 to 13, characterised in that a plate (56) which extends over the cross-section of the container and which is provided with openings (69) for the passage of gas, is arranged at a spaced distance from the bottom of the container.

15. A heat exchanger as claimed in one of Claims 9 to 14, characterised in that an inlet (45) for the cooling coil (48) also passes out of the container (35) through the cylinder (43).

16. A heat exchanger as claimed in one of Claims 9 to 15, characterised in that filters (58, 59) are arranged inside the container at the entry and/or the exit for the second fluid.

17. A heat exchanger as claimed in one of Claims 1 to 16, characterised in that the casing (39) is in the form of a basket.

18. A heat exchanger as claimed in one of Claims 9 to 17, characterised in that, on the inside of the casing (3, 39) which is in the form of a basket and/or on the outer walls of the cooling coil (48), there are arranged sealing shells (54, 55) which are connected to the casing (39) and to the container walls.

19. The use of a heat exchanger as claimed in one of Claims 1 to 18 as a reactor in methane synthesis.

20. A process for the production of a heat exchanger as claimed in one of Claims 1 to 19 comprising wound, inner and outer pipe sections (37, 38), characterised in that, in separate operating steps, the inner pipe portions (37) are wound onto a first inner tube (36), and the outer pipe portions (38) are separately wound onto a second inner tube (39), the inner diameter of which exceeds that of the outermost winding of the inner pipe portions, and that the two wound portions are subsequently inserted into one another, the respective pipe cross-sections are connected to one another, and finally the two portions are surrounded by a housing.

**Revendications**

1. Echangeur de chaleur pour deux fluides, comportant des tuyaux de passage du premier fluide disposé dans un récipient cylindrique fermé, dans lequel le volume extérieur entourant les tuyaux et prévu pour recevoir le second fluide et dans lequel toutes les entrées et sorties sont disposées dans le couvercle (2) du récipient (1), les tuyaux revenant vers l'arrière, à partir du couvercle, au voisinage de la paroi du récipient en direction de son fond, puis à partir de là, vers l'axe du récipient et à l'intérieur des tronçons tubulaires extérieurs (6a, 38) en direction du couvercle de façon à déboucher à leurs deux extrémités dans les tuyaux collecteurs (9, 9', 10, 41) passant à l'extérieur au travers du couvercle, les tronçons extérieurs et intérieurs (6a, 6b, 37, 38) des tuyaux étant séparés l'un de l'autre par une enveloppe (3, 39) qui est ouverte, sur son côté inférieur en direction du volume intérieur du récipient, caractérisé en ce que l'enveloppe (3, 39) est reliée, sur son côté supérieur, de façon étanche à un second tuyau, disposé concentriquement autour du tuyau collecteur (10, 36) et servant à la canalisation du second fluide au travers du couvercle (2) et en ce qu'il est prévu, concentriquement autour des tuyaux extérieurs collecteurs (9, 9', 41) pour le premier fluide et/ou autour du second tuyau (11, 42), en vue de former des volumes annulaires, respectivement d'autres tuyaux (12,12' et 13) qui sont reliés au volume partiel de forme annulaire (5) existant entre l'enveloppe (3, 39) et la paroi de récipient et qui sont agencés en partie sous forme de passages de traversée (12, 12') pour le second fluide.

2. Echangeur de chaleur selon la revendication 1, caractérisé en ce que l'autre tuyau (13) disposé autour du second tuyau (11) placé concentriquement autour du tuyau collecteur intérieur (10) est pourvu d'un enroulement de refroidissement (18, 46).

3. Echangeur de chaleur selon la revendication 1 ou 2, caractérisé en ce qu'une plaque de raidissage (23a, 23b, 23c, 23d, 23e), reliée rigidement au couvercle, est disposée à une certaine distance de ce couvercle (2).

4. Echangeur de chaleur selon l'une quelconque des revendications 1 à 3, caractérisé en ce que les tronçons tubulaires intérieurs (6b, 37) sont pourvus d'une chemise d'étanchéité (22a, 53) qui est reliée à l'enveloppe (3, 39).

5. Echangeur de chaleur selon l'une quelconque des revendications 1 à 4, caractérisé en ce que les tronçons tubulaires extérieurs (6a) sont pourvus d'une chemise d'étanchéité (22b) qui est reliée au côté intérieur de la paroi de récipient.

6. Echangeur de chaleur selon la revendication 5, caractérisé en ce qu'il est prévu une isolation thermique entre la chemise d'étanchéité 22b et la paroi de récipient.

7. Echangeur de chaleur selon l'une quelconque des revendications 1 à 6, caractérisé en ce que le récipient (1, 35) est pourvu au moins en partie d'une masse de remplissage.

8. Echangeur de chaleur selon la revendication 7, caractérisé en ce que la masse de remplissage contient une matière formant catalyseur.

9. Echangeur de chaleur selon l'une quelconque des revendications 1 à 8, dans lequel il n'est prévu aucun autre tuyau autour des tuyaux collecteurs extérieurs (9, 9') pour le premier fluide, caractérisé en ce qu'un cylindre (30, 43) relié au couvercle (2) et présentant au moins le diamètre des tronçons tubulaires intérieurs (6b, 37) est placé autour de l'autre tuyau (13) et comporte des passages de traversée pour le second fluide, et en ce que les tuyaux collecteurs (9", 41) reliés aux tronçons tubulaires extérieurs sont guidés vers l'extérieur au travers du cylindre (30, 43).

10. Echangeur de chaleur selon la revendication 9, caractérisé en ce que le cylindre (30, 43) est agencé sous la forme d'une suspension pour les tronçons tubulaires extérieurs (6a, 38).

11. Echangeur de chaleur selon la revendication 9, caractérisé en ce que les extrémités supérieures des tronçons tubulaires extérieurs (6a, 38) sont agencées sous la forme d'une suspension.

12. Echangeur de chaleur selon l'une quelconque des revendications 9 à 11, caractérisé en ce que le fond de récipient comporte une ouverture obturable (57).

13. Echangeur de chaleur selon l'une quelconque des revendications 9 à 12, caractérisé en ce qu'il est prévu entre la paroi de récipient et les tronçons tubulaires extérieurs (38) un enroulement de refroidissement (48), branché en parallèle avec les tronçons tubulaires extérieurs (38) et en ce que les tuyaux collecteurs (41) correspondant aux tronçons tubulaires extérieurs (38) sont reliés, par l'intermédiaire d'un tuyau de dérivation obturable (51), avec le tuyau collecteur (40) des tronçons tubulaires intérieurs (37), aussi bien l'enroulement de refroidissement (48) qu'également l'entrée des tronçons tubulaires extérieurs (38) pouvant être obturés indépendamment l'un de l'autre.

14. Echangeur de chaleur selon l'une quelconque des revendications 9 à 13, caractérisé en ce qu'une plaque (56), pourvue d'orifices de passage de gaz (69) et couvrant la section droite du récipient, est disposée à une certaine distance du fond de récipient.

15. Echangeur de chaleur selon l'une quelconque des revendications 9 à 14, caractérisé en ce qu'également un passage d'entrée (45) pour l'enroulement de refroidissement (48) sort à l'extérieur du récipient par l'intermédiaire du cylindre (43).

16. Echangeur de chaleur selon l'une quelconque des revendications 9 à 15, caractérisé en ce qu'il est prévu à l'intérieur du récipient des filtres (58, 59) à l'entrée et/ou à la sortie du second fluide.

17. Echangeur de chaleur selon l'une quelconque des revendications 1 à 16, caractérisé en ce que l'enveloppe (39) est agencée en forme de panier.

18. Echangeur de chaleur selon l'une quelconque des revendications 9 à 17, caractérisé en ce qu'il est prévu, sur le côté intérieur de l'enveloppe (3, 39) agencée en forme de panier et/ou sur la paroi extérieure de l'enroulement de refroidissement (48), des chemises d'étanchéité (54, 55) qui sont reliées à l'enveloppe (39) ou bien à la paroi de récipient.

19. Utilisation d'un échangeur de chaleur selon l'une quelconque des revendications 1 à 18 comme réacteur dans la synthèse du méthane.

20. Procédé de fabrication d'un échangeur de chaleur selon l'une quelconque des revendications 1 à 19, comportant des tronçons tubulaires enroulés intérieurs et extérieurs (37, 38), caractérisé en ce qu'on effectue dans des opérations séparées l'enroulement des tronçons tubulaires intérieurs (37) sur un premier tuyau central (36) et l'enroulement des tronçons tubulaires extérieurs (38) séparément sur un second tuyau central (39), dont le diamètre intérieur est plus grand que celui de l'enroulement complètement extérieur des tronçons tubulaires intérieurs, en ce qu'on engage l'un dans l'autre les deux tronçons enroulés, en ce qu'on relie entre elles les sections de tuyaux et en ce que les deux tronçons tubulaires sont finalement entourés d'un carter.

*Fig. 1*

Fig.2a

Fig. 2b

Fig.2c

Fig.2d

17

0 008 633

Fig. 2e

Fig. 3

Fig.4

0 008 633

Fig. 5